# FASCICULE DE BREVET EUROPEEN

(11) **EP 0 764 849 B1**
(45) Date de publication et mention de la délivrance du brevet: **20.03.2002**
(21) Numéro de dépôt: 96401973.1
(22) Date de dépôt: 17.09.1996
(51) Int. Cl.: G01N 33/573, C07K 16/38

(54) **Test immunologique ELISA pour l'inter-alpha-trypsine inhibiteur**
Immunologischer ELISA-Test für Inter-Alpha-Trypsin-Inhibitor
Immunological ELISA test for inter-alpha-trypsin inhibitor

(30) Priorité: 19.09.1995 FR 9510944
(43) Date de publication de la demande: 26.03.1997
(73) Titulaire: ASSOCIATION POUR L'ESSOR DE LA TRANSFUSION SANGUINE DANS LA REGION DU NORD, 59000 Lille (FR)
(72) Inventeur: Mizon, Jacques, 59130 Lambersart (FR); Burnouf, Thierry, 59136 Wavrin (FR)
(74) Mandataire: Lhuillier, René

(56) Documents cités:
- EP-A- 0 328 690
- US-A- 5 114 863
- CELLULAR AND MOLECULAR BIOLOGY, vol. 38, no. 4, 1992, NEW YORK, NY, pages 463-471, XP002003054 BUSINARO ET AL.: "Inter-alpha-trypsin inhibitor-related immunoreactivity ...."
- JOURNAL OF IMMUNOLOGICAL METHODS, vol. 190, 1996, AMSTERDAM, pages 61-70, XP000196043 MIZON ET AL.: "Development of an enzyme-linked immunosorbent assay for human plasma inter-alpha-trypsin inhibitor ...."
- DELVES P.J. AND ROITT I.M.: 'ENCYCLOPEDIA IMMUNOLOGY, SECOND EDITION', ACADEMIC PRESS * page 1252 - page 1257 *

## Description

L'invention concerne un test immunologique pour la détection et le dosage spécifiques de l'ITI ainsi que le procédé de préparation d'immunoglobulines spécifiques qui permettent de piéger l'ITI "en sandwich" et qui rendent ledit test possible.

L'inter-α-trypsine inhibiteur (ITI) est un inhibiteur de sérine-protéases du plasma humain. Outre la trypsine et la chymotrypsine, il inhibe différentes protéinases impliquées dans les états inflammatoires, comme l'élastase leucocytaire, la cathepsine G et la plasmine. Cependant sa capacité inhibitrice est faible comparée à celle d'autres inhibiteurs de protéases plasmatiques comme l'α₁-protéinase inhibiteur (α₁ PI anciennement appelé α₁ antitrypsine), l'α₁-antichymotrypsine, l'α₂- antiplasmine et l'α₂- macroglobuline. Sa vraie fonction physiologique n'est donc pas encore élucidée.

Des études de structure ont montré que le plasma contient plusieurs molécules voisines de l'ITI et qui constituent une "superfamille" dont les 4 membres sont bien caractérisés :
1) la bikunine (BK, PM 30 000 d.), responsable de l'activité antiprotéase, est présente à l'état libre dans le plasma en très faible quantité ( environ 6,4 mg/l);
2) l'ITI (PM 220 000d) constitué d'une molécule de bikunine liée de manière covalente à 2 chaînes lourdes (H₁ et H₂) par une chaîne de glycosaminoglycane, est le principal constituant de la famille ;
3) le pré-α trypsine inhibiteur (paI, PM 130 000 d) est constitué d'une molécule de bikunine liée de la même manière à une troisième chaîne lourde (H3) ;
4) la quatrième molécule, l'inter-α-like inhibiteur(I αLI, PM 125 000 d) est constitué de bikunine associée avec la chaîne lourde H₂.

On isole encore, à partir du plasma, des glycoprotéines d'un poids moléculaire d'environ 120 000d qui présentent une homologie significative avec la région N-terminale de la chaîne H₁ (40%), H₂ (39%) et H₃ (47%) ce qui indique que des consituants proches des chaînes lourdes de l'ITI peuvent être sécrétés dans le plasma indépendamment de la bikunine.

Il est maintenant démontré que les molécules de la famille de l'ITI sont codées par 5 gènes distincts et régulés indépendamment (Daveau et al. 1993). L'étude fine de leurs métabolismes ne sera possible que lorsqu'on disposera de moyens de détermination spécifiques de chacune de ces molécules. C'est un des objectifs de la présente invention.

En effet, les méthodes de détection de l'ITI connues actuellement ne donnent pas satisfaction, soit parce qu'elles sont non spécifiques de l'ITI soit parce qu'elles sont imprécises.
- Hochstrasser et al. (1977) déterminent l'activité inhibitrice de la bikunine libérée par protéolyse des protéines sériques dénaturées.
- En absence de test quantitatif de l'ITI, plusieurs méthodes immunologiques ont été utilisées, comme l'électroimmunodiffusion (Salier et al. 1983, Chawla et al. 1984) ou l'ELISA (Businaro et al. 1992). Mais comme il n'existe pas d'anticorps qui différencient l'ITI des autres molécules de la même famille, les résultats obtenus ne sont pas fiables. En particulier Odum (1990) et Gressier et al. (1990) ont montré que même l'immunoélectrophorèse croisée ne sépare pas bien l'ITI de ses dérivés et ne mesure pas spécifiquement l'ITI.

L'ITI n'est clairement séparé que par électrophorèse sur gel de polyacrylamide-SDS (SDS-PAGE) (Laroui et al. 1988) et celle-ci peut être suivie d'une analyse par immunoempreinte pour obtenir une détermination semi-quantitative (Odum et al. 1990 ; Daveau et al. 1993). Ce procédé est trop complexe pour être standardisé et utilisé en routine.

Pour combler cette absence de moyen de mesure à la fois spécifique et d'utilisation simple, la Demanderesse a développé une méthode de mesure spécifique de l'ITI natif. Dans ce but, elle a préparé des immunoglobulines (anticorps polyclonaux) spécifiques de chacune des 2 chaînes H₁ et H₂ de l'ITI pour mettre au point un test ELISA "en sandwich" permettant la détection et le dosage spécifiques de l'ITI.

Ces immunoglobulines spécifiques sont obtenues par immunisation d'animaux (des lapins par exemple) avec des préparations hautement purifiées de chacune des 2 molécules H₁ et H₂. Celles-ci sont obtenues par chromatographie d'échange d'ions après dissociation alcaline d'une fraction plasmatique enrichie en ITI. Les immunoglobulines selon l'invention sont purifiées à partir des immunsérums par élimination des immunoglobulines contaminantes (dirigées contre les autres fragments de l'ITI) par adsorption sur lesdits contaminants purifiés et couplés sur Sepharose® . Plus précisément:
- les immunoglobulines spécifiques anti-H₁ sont débarrassées des contaminants anti- H₂ et anti-bikunine par adsorption de ceux-ci sur une préparation de protéine H₂ purifiée et immobilisée sur Sépharose® par couplage au CNBr puis sur une préparation de bikunine immobilisée sur Sepharose® par couplage au CNBr.
- les immunoglobulines anti-H₂ sont débarrassées des contaminants anti-H₁ par adsorption de ceux-ci sur une préparation de protéine H₁ purifiée et immobilisée sur Sepharose® par couplage au CNBr.

L'obtention des immunoglobulines spécifiques de H₁ ou de H₂ était le préalable nécessaire à la mise au point du test immunologique selon l'invention. Plus précisément, celui-ci comprend l'utilisation, comme réactifs "en sandwich", de la combinaison d'immunoglobulines spécifiques de l'une des 2 chaînes lourdes de l'ITI fixées à un support et d'immunoglobulines spécifiques de l'autre chaîne lourde de l'ITI couplées à un marqueur de type biotine comme solution révélatrice de la présence d'ITI.

L'utilisation de molécules biotinylées en immunologie est largement répandue à cause de leur facilité de détection qui repose sur la forte affinité de la biotine pour l'avidine et la streptavidine. Ces dernières sont utilisées sous forme complexée à une enzyme qui donne lieu à une réaction colorée et quantifiable, en présence de son substrat. Ainsi la streptavidine couplée à la peroxydase ou à la phosphatase alcaline est couramment utilisée. Selon un mode préféré de réalisation du test, on utilise les immunoglobulines spécifiques de la chaîne H₁ fixées au support et celles spécifiques de la chaîne H₂ comme solution révélatrice.

Le support est du type plaque multipuits pour microtitration, disponible chez plusieurs fournisseurs (Nunc® par exemple), qui permet une mesure de réactions colorées en appareil automatique. D'autres types de supports peuvent être utilisés dans le même but, comme des microtubes associés par moulage dans une plaquette, du type destiné à l'immunohématologie, ou tout autre support permettant la fixation des immunoglobulines et la lecture de la réaction ultérieure.

La mise en oeuvre du test selon l'invention comprend :
- la mise en contact d'un prélèvement sanguin ou d'un échantillon dont on veut déterminer le contenu en ITI avec les immunoglobulines fixées,
- des lavages pour éliminer les constituants non adsorbés,
- et la révélation de l'ITI adsorbé sur les premières immunoglobulines par la solution des secondes immunoglobulines et l'amplification de ladite révélation par une cascade de réactions connue, de type biotine-streptavidine-peroxydase ou -phosphatase alcaline.
L'invention s'étend également à la présentation du test sous forme de kit prêt à l'emploi et comprenant :
- un support de type plaque à puits multiples pour microtitrages, puits sur lesquels sont adsorbées les immunoglobulines anti-H₁
- une préparation des immunoglobulines anti-H₂ marquées à la biotine.

Les exemples suivants illustrent l'invention sans toutefois en limiter la portée.

### Exemple 1. Isolement des 3 chaînes constituant l'ITI

L'ITI est obtenu comme un sous-produit du procédé de fractionnement du plasma destiné à la purification du Facteur IX (Michalski et al. 1994).

Les chaînes H₁ et H₂ sont isolées par chromatographie d'échange d'ions après traitement de dissociation alcaline dans les conditions suivantes : on mélange l'ITI, 80 mg dans 100 ml d'eau, avec un volume égal de Na₂CO₃ 0,1M. Le pH est ajusté à 12,5 avec du NaOH 10M et le mélange est agité pendant 10 minutes à température ordinaire puis neutralisé avec de l'HCl 2M. Après une nuit de dialyse contre du tampon Tris 0,02M/HCl, EDTA 4mM, pH 7,6, l'ITI dissocié est chargé sur une colonne de Q-Sepharose ® FF, dans le même tampon, à un débit de 100 ml/h. Après lavage de la colonne pendant 30 min avec le même tampon, les protéines sont éluées par l'application d'un gradient linéaire de NaCl de 0 à 0,5M, dans le même tampon.
Les fractions sont récoltées séparément et analysées par SDS 10% /PAGE pour récolter les fractions contenant les chaînes H₁ et H₂.

La bikunine est également isolée à partir d'une préparation d'ITI partiellement purifiée, contenant 1g de protéine par 100 ml, et dissociée en conditions alcalines à pH 12 pendant 30 minutes. On ajoute ensuite de l'acide perchlorique à 35 % (5ml pour 25 ml du mélange réactionnel) et on incube pendant 1 heure à 4°C. Après centrifugation à 18 000 rpm pendant 15 minutes à 4°C le surnageant est amené à pH7 par addition de KOH 5M et il est récupéré.

### Exemple 2 Immunisation des animaux et obtention des anticorps

Des lapins sont immunisés par injections intracutanées à plusieurs endroits.
Chaque antigène est injecté à raison de 1 mg/ml en solution saline de tampon phosphate 0,1M (PBS), pH 7,4, émulsifiée avec de l'adjuvant de Freund incomplet (selon un protocole classique décrit par Vaitukaitis et al. 1971)

Les immunsérums bruts sont purifiés sur les protéines correspondantes H₁ ou H₂ immobilisées sur Sepharose® 4B selon le protocole suivant : chacune des protéines (H₁ et H₂) est dialysée 1 nuit contre du tampon NaHCO₃ 0,1M, NaCl 0,5M à pH 8,3, à raison de 50 mg dans 10ml de tampon, puis couplée à 10ml de Sepharose ® activé au CNBr.
5 ml d'immunsérum anti-H₁ ou anti-H₂ sont dilués (1/5 v/v)
dans du tampon Tris 0,75 mM, NaCl 0,15M, pH 7,8 et dialysés 1 nuit à 4°C contre le même tampon. Ils sont ensuite incubés en présence des antigènes H₂ ou H₁ couplés aux billes de Sepharose ®, à 4°C sous agitation douce, puis disposés dans des colonnes. Les immunoglobulines spécifiques non fixées sur le support sont récupérées par lavage avec le tampon d'équilibrage. La colonne est ensuite régénérée par du tampon Gly-HCl 100 mM, pH 2,7.

L'immunsérum anti-H₁ est, en outre, adsorbé sur de la bikunine-Sepharose®, suivant le même protocole, parce qu'on a observé qu'il était fortement contaminé par de l'anti-bikunine,

Les fractions d'immunoglobulines des immunsérums anti-H₁ et anti-H₂ sont purifiées sur protéine A-Sepharose® et concentrées en échantillons de 10 ml, selon un protocole classique et les instructions du fournisseur (Pharmacia-Biotech)

### Exemple 3. Mise en évidence de la spécificité des immunoglobulines

La spécificité des immunoglobulines purifiées a été déterminée par ELISA et par immunoempreintes après électrophorèse sur gel de polyacrylamide.

### 1) ELISA

On utilise des microplaques multipuits (par exemple fournies par Nunc®). L'antigène (H₁, H₂, BK ou ITI) est adsorbé sur les puits par un contact de 15 h à 4°C avec 100 µl par puits d'une solution à 1,3 mg d'antigène/l de tampon d'adsorption (NaHCO₃ 20 mM, pH 9,5).
Après 4 lavages en tampon PBS 0,1M, pH 7,4 - Tween 20 0,05%, on ajoute 150 µl de sérumalbumine bovine par puits, pour bloquer les sites de liaison résiduels.
Ensuite, on ajoute 100 µl de sérum brut ou d'immunoglobuline purifiée dans chaque puits et on incube 60 min à 37°C. Après lavage en PBS-Tween,on ajoute 100 µl par puits de conjugué (immunoglobulines de chèvre, anti-immunoglobulines de lapin, marquées à la peroxydase, à 1/10000 en tampon PBS-Tween-sérumalbumine) et on incube 30 min à 37°C. Après 4 lavages en tampon PBS-Tween, on révèle le résultat par addition de 100 µl d'orthophénylènediamine (1,4 mg/ml en tampon phosphate-citrate 0,1M, pH 5,5 et 0,003 % H₂O₂) et incubation pendant 30 min à température ordinaire. La réaction est arrêtée par l'addition de 100 µl d'HCl 1M. La lecture est effectuée dans un lecteur de microplaques (Dynatech ® MR 700) par détermination de l'absorbance à 490 et 630 nm.

Dans ce test, les immunsérums bruts anti-H₁ et anti-H₂ réagissent tous les deux avec les deux chaînes H₁ et H₂ fixées dans les microplaques. De plus l'anti-H₁ réagit fortement avec la bikunine.
Par contre, après immunoadsorption, selon le protocole décrit dans l'exemple 2, chaque immunoglobuline anti-H₁ ou anti-H₂ réagit exclusivement avec l'antigène (H₁ ou H₂) contre lequel elle a été induite et sélectionnée. Les deux types d'immunoglobulines (anti-H₁ et anti-H₂) réagissent également avec l'ITI intact.

### 2 Immunoempreintes après électrophorèse sur gel de polyacrylamide

La spécificité des immunoglobulines a été confirmée par détection de l'ITI natif ou digéré par la chondroïtinase, après électrophorèse sur gel de polyacrylamide (SDS-PAGE) en conditions non réductrices ou réductrices, puis transfert sur nitrocellulose et détection immunologique.
. L'ITI natif est révélé par les immunoglobulines polyspécifiques anti-ITI et par les immunoglobulines purifiées anti-H₁ et anti-H₂.
. La chaîne lourde H₁ libérée par la digestion par la chondroïtinase est révélée par les immunoglobulines anti-ITI et par les immunoglobulines purifiées anti-H₁ mais pas par les anti-H₂.
. De même, la chaîne lourde H₂ est révélée par les anti-ITI et les anti-H₂ mais pas par les anti-H₁.

D'autre part, un échantillon de plasma humain a été analysé dans les mêmes conditions de SDS-PAGE, avant ou après digestion par la chondroïtinase et après immunoadsorption sur de l'anti-H₂ immobilisé sur billes de Sepharose ® (selon le protocole du fournisseur) ou sur de l'anti-αPI immobilisé (témoin négatif).L'adsorption est réalisée sous légère agitation pendant une nuit à 4°C. Ensuite après 10 minutes de centrifugation à 1500rpm les surnageants sont récupérés et soumis à l'électrophorèse (SDS-PAGE). Après électrophorèse, la disparition des bandes correspondant à l'ITI et à la chaîne H₂ mais pas celle correspondant à H₁ démontre qu'elles ont été totalement adsorbées sur les billes recouvertes d'anti-H₂.

Ces résultats sont en accord avec la stucture proposée pour l'ITI et décrite plus haut, c'est-à-dire une chaîne lourde H₁ et une chaîne lourde H₂ liées de manière covalente à la bikunine.

### Exemple 4. Mise au point d'un test ELISA spécifique de l'ITI

Les résultats décrits dans l'exemple précédent ont permis à la Demanderesse de développer un test ELISA original permettant la détection spécifique de l'ITI. Le principe est de piéger l'ITI entre des immunoglobulines anti-H₁ immobilisées et des immunoglobulines anti-H₂ libres et biotinylées qui seront ensuite révélées par une réaction colorée.

Des microplaques à puits multiples sont recouvertes d'anti-H₁ dans les conditions décrites plus haut (Exemple 3,1).
Les immunoglobulines anti-H₂ sont biotinylées en présence de 1 mg de NHS-LC-biotine (Pierce) pour 10 mg de protéines, selon les instructions du fournisseur.
Après divers essais préliminaires, les conditions optimales retenues sont :
- 10 mg/l d'immunoglobulines anti-H1, pour recouvrir les puits (avec 100 µl par puits)
- 5 mg/l d'immunoglobulines anti-H2 biotinylées pour révéler l'ITI adsorbée sur l'anti-H1.

On établit une courbe standard (absorbance en fonction de la concentration) avec des concentrations d'ITI de 12,5 à 200 µg/l.
On utilise 100 µl d'échantillon par puits. Après 1 heure d'incubation à 37°C suivie de 4 lavages en tampon PBS-Tween, on révèle les protéines fixées par l'addition de 100 µl par puits d'anti-H2 biotinylées.
Après 30 min d'incubation et 4 lavages, on mesure la quantité de biotine fixée.
De manière classique, la biotine est détectée par une incubation de 1 heure à 37°C en présence de streptavidine marquée à la peroxydase ou à la phosphatase alcaline. La réaction à la peroxydase a été décrite dans l'exemple 3,1. La phosphatase alcaline est visualisée par l'addition de para-nitrophényl-phosphate (1 mg/ml - Sigma® ) en tampon Tris-HCl 0,1 M pH 9, NaCl 0,1 M, MgCl₂ 5 mM et hydrolyse pendant 1 heure. La densité optique des puits est mesurée à 405 nm, par exemple à l'aide d'un lecteur automatique d'ELISA.

La sensibilité du test, c'est-à-dire la dose minimale détectée comme statistiquement positive par rapport au tampon témoin est de 2,35 µg/l ± 0,38.

La spécificité du test est démontrée par l'absence totale de détection de l'ITI digéré par la chondroïtinase.
Des dilutions en série d'échantillons de plasma donnent des résultats pratiquement parallèles à la courbe standard. Il n'y a donc pas d'interférences dues à d'autres protéines plasmatiques, ce qui confirme la grande spécificité du test.

La concentration plasmatique en ITI a été mesurée dans 30 prélèvements de sujets en bonne santé ; la concentration moyenne est de 241,5 mg/l avec comme extrêmes 145,5 et 506 mg/l.

Le test ELISA ainsi mis au point peut aisément être présenté sous forme de " kit" prêt à l'emploi comprenant :
- un support de type microplaque multipuits sur lequel sont adsorbées les immunoglobulines anti-H1,
- une préparation d'immunoglobulines anti-H2 biotinylées (ou marquées de toute autre manière utilisable en immunodétection)
- les tampons et réactifs nécessaires à la révélation de la réaction (streptavidine couplée à un marqueur donnant lieu à une réaction colorée, dans le cas de la révélation de la biotine)
- et un échantillon standard d'ITI pour étalonner le kit.

### Références bibliographiques

Businaro, R, Leali, F.M.T., De Renzis, G., Pompeli, E., Pagliari, G., Menghi, G. et Fumagali, L. (1992)
Cell Mol. Biol., 38, 463.
Byrjalsen, I., Hansen-Nord, G. and Odum, L. (1986)
Clin. Chim. Acta, 161, 59
Chawla, R. K., Raush, D.J., Miller, F.W., Vogler, R.W. et Lawson, D.H. (1984)
Cancer Res., 44,2718
Daveau, M.,Rouet, P.,Scotte, M. Hiron, M., Lebreton, J.P. et Salier, J.P. (1993)
Biochem.J., 292, 485
Gressier, B., Balduyk, M., Mizon, C. et Mizon, J. (1990) Biol. Chem. Hoppe-Seyler,371,865
Hochstrasser, K., Niebel, J., Feuth, H et Lempart, K. (1977) Klin, Wschr., 55,337
Laroui, S., Balduyk, M., Mizon, C., Selloum, L. et Mizon, J.(1988)
Biochim. Biophys. Acta, 953, 263
Michalski, C., Piva, F., Balduyck, M., Mizon, C., Burnouf, T., Huart, J.J. et Mizon, J. (1994)
Vox Sang., 67, 329
Odum, L. (1990)
Biol. Chem. Hoppe-Seyler, 371, 1153
Salier, J.P. (1990)
Trends Biochem. Sci, 15, 435
Vaitukaitis, J., Robbins, J.B., Nieschlag, E. et Ross, G.T. (1971)
J. Clin. Endocr., 33, 988

## Revendications

1. Test immunologique pour la détection et le dosage spécifique de l'ITI, **caractérisé en ce qu'**il comprend l'utilisation, comme réactifs "en sandwich", de la combinaison d'immunoglobulines spécifiques de l'une des 2 chaînes lourdes de l'ITI fixées à un support et d'immunoglobulines spécifiques de l'autre chaîne lourde de l'ITI couplées à un marqueur de type biotine, comme solution révélatrice de la présence d'ITI.

2. Test selon la revendication 1 **caractérisé en ce que** les immunoglobulines spécifiques de la chaîne H1 de l'ITI sont fixées au support et que les immunoglobulines spécifiques de la chaîne H2 sont utilisées comme solution révélatrice.

3. Test selon la revendication 1 ou 2, **caractérisé en ce que** sa mise en oeuvre comprend :
- la mise en contact d'un prélèvement sanguin ou d'un échantillon dont on veut déterminer le contenu en ITI avec les immunoglobulines fixées,
- des lavages pour éliminer les constituants non adsorbés,
- et la révélation de l'ITI adsorbée sur les premières immunoglobulines par la solution des secondes immunoglobulines et l'amplification de ladite révélation par une cascade de réactions connue, de type biotine-streptavidine-peroxydase ou -phosphatase alcaline.

4. Procédé de préparation des immunoglobulines spécifiques de la chaîne lourde H1 de l'ITI destinées au test selon l'une quelconque des revendications 1 à 3, **caractérisé en ce qu'**il comprend :
- l'immunisation d'un animal avec une préparation de H1 de haute pureté,
- la récupération de l'immunserum brut,
- et la purification des immunoglobulines spécifiques anti-H1 par élimination des immunoglobulines contaminantes, en particulier des anti-H2 et des anti-bikunine, par adsorption sur une préparation de protéine H2 immobilisée sur Sepharose® par couplage au CNBr puis sur une préparation de bikunine immobilisée sur Sepharose® par couplage au CNBr.

5. Procédé de préparation des immunoglobulines spécifiques de la chaîne lourde H2 de l'ITI destinées au test selon l'une quelconque des revendications 1 à 3, **caractérisé en ce qu'**il comprend :
- l'immunisation d'un animal avec une préparation de H2 de haute pureté,
- la récupération de l'immunserum brut,
- et la purification des immunoglobulines spécifiques anti-H2 par élimination des immunoglobulines contaminantes, en particulier des anti-H1, par adsorption sur une préparation de protéine H1 immobilisée sur Sepharose® par couplage au CNBr.

6. Kit pour la réalisation du test selon l'une des revendications 1 à 3, **caractérisé en ce qu'**il comprend :
- les immunoglobulines anti-H1 obtenues par le procédé selon 1a revendication 4, adsorbées sur un support de type plaque à puits multiples pour microtitrages,
- et une préparation des immunoglobulines anti-H2 obtenues par le procédé selon la revendication 5, marquées à la biotine.

## Patentansprüche

1. Immunologischer Test zum Nachweis und zur spezifischen Bestimmung des ITI, **dadurch gekennzeichnet, dass** er die Verwendung, als "Sandwich"-Reagenzien, der Kombination von für eine der zwei schweren Ketten des ITI spezifischen Immunglobulinen, die an einen Träger gebunden sind, und von für die andere schwere Kette des ITI spezifischen Immunglobulinen, die an einen Biotin-Marker gekoppelt sind, als Lösung zum Nachweis der Anwesendheit von ITI umfasst.

2. Test nach Anspruch 1, **dadurch gekennzeichnet, dass** die für die Kette H1 des ITI spezifischen Immunglobuline an den Träger gebunden sind und dass die für die Kette H2 spezifischen Immunglobuline als Nachweislösung verwendet werden.

3. Test nach Anspruch 1 oder 2, **dadurch gekennzeichnet, dass** seine Durchführung umfasst :
- das Inkontaktbringen einer Blutprobe oder einer Probe, deren Gehalt an ITI man bestimmen will, mit den gebundenen Immunglobulinen,
- Waschungen, um die nichtadsorbierten Bestandteile zu beseitigen,
- und den Nachweis des an den ersten Immunglobulinen adsorbierten ITI durch die Lösung der zweiten Immunglobuline und die Amplifikation dieses Nachweises durch eine bekannte Kaskade von Reaktionen des Typs Biotin-Streptavidin-Peroxidase oder alkalische -Phosphatase.

4. Verfahren zur Herstellung von für die schwere Kette H1 des ITI spezifischen Immunglobulinen, die für den Test nach irgendeinem der Ansprüche 1 bis 3 bestimmt sind, **dadurch gekennzeichnet, dass** es umfasst :
- die Immunisierung eines Tieres mit einer Zubereitung von H1 mit hoher Reinheit,
- die Gewinnung von rohem Immunserum,
- und die Reinigung der anti-H1 spezifischen Immunglobuline durch Beseitigung von verseuchenden Immunglobulinen, insbesondere von anti-H2 und anti-Bikunin, durch Adsorption an einer Zubereitung von Protein H2, das durch Kopplung mittels CNBr an Sepharose® immobilisiert ist, und anschließend an einer Zubereitung von Bikunin, das durch Kopplung mittels CNBr an Sepharose® immobilisiert ist.

5. Verfahren zur Herstellung von für die schwere Kette H2 des ITI spezifischen Immunglobulinen, die für den Test nach irgendeinem der Ansprüche 1 bis 3 bestimmt sind, **dadurch gekennzeichnet, dass** es umfasst :
- die Immunisierung eines Tieres mit einer Zubereitung von H2 mit hoher Reinheit,
- die Gewinnung von rohem Immunserum,
- und die Reinigung der anti-H2 spezifischen Immunglobuline durch Beseitigung von verseuchenden Immunglobulinen, insbesondere von anti-H1, durch Adsorption an einer Zubereitung von Protein H1, das durch Kopplung mittels CNBr an Sepharose® immobilisiert ist.

6. Kit für Durchführung des Tests nach einem der Ansprüche 1 bis 3, **dadurch gekennzeichnet, dass** es umfasst :
- die anti-H1-Immunglobuline, die durch das Verfahren nach Anspruch 4 erhalten werden und an einen Träger von der Art einer Platte mit mehreren Vertiefungen für Mikrotitration adsorbiert sind,
- und eine Zubereitung von anti-H2-Immunglobulinen, die durch Verfahren nach Anspruch 5 erhalten werden und mit Biotin markiert sind.

## Claims

1. Immunoassay for detection and specific analytical assay of ITI, wherein it comprises the use, as "sandwich" reagents, of the combination of specific immunoglobulins from one of the two ITI heavy chains fixed on a support and of specific immunoglobulins of the other ITI heavy chain linked with a biotin marker, as tracer solution of the presence of ITI.

2. Assay according to claim 1 wherein the specific immunoglobulins of the ITI H1 chain are fixed on the support and that the specific immunoglobulins of the H2 chain are used as tracer solution.

3. Assay according to claim 1 or 2, wherein its implementation comprises :
- putting into contact of a blood sample or of a sample the ITI content of which we want to determine with the fixed immunoglobulins,
- washings to eliminate non adsorbed constituents,
- and revelation of the ITI adsorbed on the first immunoglobulins by the solution of the second immunoglobulins and amplification of said revelation by a cascade of known biotin-streptavidin-peroxydase or alcalin
- phosphatase type reactions.

4. Method for preparing specific immunoglobulins of the ITI H1 heavy chain intended for the assay according to any of claims 1 to 3, wherein it comprises :
- immunisation of an animal with a high purity H1 preparation,
- recovery of the crude immunserum,
- and purification of the specific anti-H1 immunoglobulins by elimination of contaminating immunoglobulins, in particular anti-H2 and anti-bikunine, by adsorption on a H2 protein preparation immobilised on Sepharose® by CNBr coupling then on a bikunine preparation immobilised on Sepharose® by CNBr coupling.

5. Method for preparing specific immunoglobulins of the ITI H2 heavy chain intended for the assay according to any of claims 1 to 3, wherein it comprises :
- immunisation of an animal with a high purity H2 preparation,
- recovery of the crude immunserum,
- and purification of the specific anti-H2 immunoglobulins by elimination of contaminating immunoglobulins, in particular anti-H1, by adsorption on a H1 protein preparation immobilised on Sepharose® by CNBr coupling.

6. Kit for carrying out the assay according to any of claims 1 to 3, wherein it comprises :
- the anti-H1 immunoglobulins obtained by the method according to claim 4, adsorbed on a support of multiple wells plate type for microtitration,
- and an anti-H2 immunoglobulins preparation obtained by the method according to claim 5, traced with biotin.
